# EUROPEAN PATENT APPLICATION

(11) **EP 4 403 620 A1**
(43) Date of publication of application: **24.07.2024**
(21) Application number: 23152056.0
(22) Date of filing: 17.01.2023
(51) Int. Cl.: C12M 1/36

(54) **METHOD FOR PERFORMING AND SUPPORTING A BIOPROCESS PERFORMED ON A LIQUID IMMUNE OR NAIVE CELL CULTURE**

(71) Applicant: The Automation Partnership (Cambridge) Ltd., Hertfordshire SG8 5WY (GB)
(72) Inventor: Stacey, Adrian, Cambridge CB4 1FY (GB); Prouté, Fanny, 37083 Göttingen (DE); Betts, John, London N4 4BX (GB); Pilgrim, James, Cambridge CB5 8PJ (GB)
(74) Representative: Gottschald Patentanwälte Partnerschaft mbB

(57) **Abstract**

The invention relates to a method for performing and supporting a bioprocess performed on a liquid immune or naive cell culture, wherein the bioprocess is performed on the cell culture to obtain a processed cell culture, wherein the processed cell culture is destined for autologous or allogenic cell therapy, wherein the bioprocess is performed by an integrated bioprocessing system (1) performing multiple unit operations on the cell culture, wherein a virtual identity (5) is assigned to the cell culture, wherein the virtual identity (5) is stored by a control unit (7) in a local memory (8), wherein the control unit (7) gathers data about the surroundings of the cell culture while the cell culture is processed by the integrated bioprocessing system (1) and wherein the gathered data is stored assigned to the virtual identity (5). It is proposed, that the cell culture is transported from a first location to a second location and that the control unit (7) including the memory is transported with the cell culture from the first location to the second location.

## Description

The present invention relates to a method for performing and supporting a bioprocess performed on a liquid immune or naive cell culture according to the general part of claim 1 and to a control unit adapted to gather data according to claim 21.

The term "bioprocess" presently represents a biotechnological process, in particular a biotechnological process involving the use of immune cell cultures or naive cell cultures, in particular stem cell cultures. One or more processing steps might be performed on each cell culture. Hence, a bioprocess in this sense might refer to a manufacturing process that involves a sequence of processing steps performed on a cell culture which ultimately will lead to a final product.

Exemplarily, the bioprocess may be in the area of cell and gene therapy, for example to manufacture autologous T cells that are modified to express a chimeric antigen receptor (CAR). These cells might be used for the treatment of various types of hematologic malignancies, including different types of leukemia (blood cancer). Other cell therapies based on naive cells, in particular stem cells and their derivates are also of interest.

For biotechnological processes involving the use of liquid immune or naive cell cultures the starting material usually is quite heterogeneous regarding its composition, for example each donor or patient may be in a different condition (e.g., in terms of disease progression, genetic make-up or the immune system history). Therefore, certain steps of the bioprocess need to be flexibly adapted and tailored to each individual cell culture. Furthermore, a bioprocess involving the genetic modification of an immune cell culture will require a sequence of processing steps that is different from the sequence of processing steps required in a bioprocess not involving the genetic modification of an immune cell culture.

Hence, depending on the features of the cell culture and the bioprocess to be performed, various parameters, including for example the type, sequence or configuration of processing steps performed on each immune cell culture will need to be adjusted to the individual features of the cell culture. Further, the cell cultures are typically processed by different devices or need to be transferred between different locations to be processed. This may require an operator to pre-configure or adjust different devices to each cell culture, including the preconfig-uration or reconfiguration of single-use components like tubes or tube-sets. Accordingly, different tubes may be connected differently or need to be installed into certain device components manually when performing the bioprocess.

In many bioprocesses, including the area of cell and gene therapy which may comprise allogenic or autologous production of genetically modified immune cells, compliance of the process with regulatory demands is important. To ensure the safety of the product, the manufacturing process is typically highly regulated by regulatory authorities.

Bioprocesses, in particular production processes in the field of cell and gene therapy, usually involve a plurality of process steps. Accordingly, the cell culture is usually transferred, manually or automatically, between different devices. Further, the cell culture may also be transferred from and to different liquid containers and usually numerous reagents and equipment are utilized. The resulting data, including the type and quantity of materials used, process conditions and any deviations must be documented, and the documentation must be assigned to the final product in a suitable manner.

Recently, more and more integrated bioprocessing systems are proposed. Such integrated bioprocessing systems allow simultaneous performance of several bioprocesses on different cell cultures, wherein the bioprocesses or rather the processing steps to be performed can be adjusted according to the respective cell culture. Known integrated bioprocessing systems are centralized systems which comprise central control units and central documentation and workflow management.

It is a challenge to improve the documentation of bioprocesses and further ensure that the cell cultures are not subjected to adverse conditions. Losing a cell culture to a system error can have very severe consequences for the patient.

The invention is based on the problem of improving the known methods for performing and supporting a bioprocess performed on a liquid immune or naive cell culture such that the safety of the cell culture is further increased.

The above-noted object is solved by the features of the characterizing part of claim 1.

The main realization of the present invention is that while an integrated bioprocessing system is a centralized system with usually many centralized functions, the cell culture needs to enter the integrated bioprocessing system one or more times and leave the integrated bioprocessing system one more times. In addition to that, some integrated bioprocessing systems may be, to some extent, modularized such that in extreme cases, the integrated bioprocessing system may not know which bioprocess it is performing. For example, cartridges configured for the performance of the bioprocess may be driven and/or transported by the integrated bioprocessing system without the necessity of the integrated bioprocessing system fully knowing what it is driving and/or transporting.

Further, centralized connections may break down, sensors may fail and generally, mistakes can be made. Gathering and storing and possibly processing data in a decentralized manner focused on the cell culture and physically following the cell culture allows a different, possibly redundant, safety check and documentation. In particular, providing an independent control unit that checks whether the integrated bioprocessing system is performing the bioprocess correctly and/or whether the ambient conditions of the cell culture are within specification, from the viewpoint of a single cell culture instead of a central viewpoint, increases the chances of detecting and/or preventing faults.

For this, assigning a virtual identity to a cell culture and saving this virtual identity in a local memory of a control unit near to the cell culture is proposed.

In detail, it is proposed that the cell culture is transported from a first location to a second location and that the control unit including the memory is transported with the cell culture from the first location to the second location.

In an embodiment according to claim 2, the control unit may be placed at the container and may be transported together with the container. A separate handling of the control unit can then be avoided. A particularly easy handling is achieved if the control unit is connected to the container in such a way that by transporting the container, the control unit is also transported. For example, the control unit may be part of the container or put in the container.

Possible implementations of integrated bioprocessing systems may require the transport of the cell culture between a first container and a second container. In an embodiment according to claim 3, the virtual identity may be transported together with the cell culture, either digitally by transferring the data or mechanically by transferring the control unit.

Claim 4 relates to the transfer of the virtual identity being triggered and possibly validated by the integrated bioprocessing system. This further emphasizes the possible redundancy of the proposed solution leading to more security and shows the partial intertwining of the control unit with the integrated bioprocessing system.

The necessity of monitoring the surroundings of the cell culture may not be present all the time, or it may not be necessary to have a redundant monitoring all the time. It may be the case that the integrated bioprocessing system transports the cell culture between different locations through an intermediate container. It is then possible to reduce complexity and cost by transporting the virtual identity directly from the first container to the second container, bypassing the intermediate container (claim 5).

Embodiments according to claim 6 relate to securing a component of the control unit or the gathered data for example by encryption. In particular, it may be the case that the control unit signs the gathered data. By having a physical component assigned to the cell culture and securing this physical component crypto-graphically and/or physically, intentional and unintentional altering of data can be made more unlikely.

An embodiment according to claim 7 relates to a sensor of the control unit.

A preferred embodiment according to claim 8 includes the control unit gathering data about a unit operation station of the integrated bioprocessing system. The control unit may provide an independent validation of the performance of the bioprocess and may ensure that the correct bioprocess is performed. Claim 9 names possible details of the connection between the control unit and the unit operation station. In a particularly interesting case, an interface between the unit operation station and the container is used for the connection.

The gathered data may be stored locally by the control unit or at a central location (claim 10).

Preferred embodiments according to claim 11 deal with transporting the control unit in and/or out of the integrated bioprocessing system to monitor the cell culture also outside the integrated bioprocessing system, possibly on its full journey (claim 12).

The already named redundancy is an object of an embodiment according to claim 13.

Particular challenges arise, when a user needs to perform steps of the bioprocess and when the access to information may be time-critical and decisions need to be made within a certain time window to ensure compliance with regulatory demands or, especially for digital approaches, when the communication to a central management or database is lost.

In a highly regulated process, as stated above, any operator involvement required poses a threat to the product, because manual steps to be performed may be error-prone, for example if tube connections are established wrongly, or the sterility of the product may become endangered, if the wrong components are handled. However, complex processes can only be automated to a certain degree and critical decisions should ultimately be made by knowledgeable operators. Accordingly, to mitigate these risks, operators need to be trained extensively, a variety of measurements need to be taken, and an extensive documentation is mandatory to ensure the safety of the product. In this regard, user support is not just a question of working efficiently, but can also be a decisive step towards product quality and safety. This issue is dealt with in claim 14.

Details of ensuring the authenticity of the gathered data or parts of it are detailed in claims 15 and 16.

The control unit, according to claim 17, may know about a workflow performed during the bioprocess and may therefore be able to detect deviations from this workflow. The control unit may also, at least partially, cause this workflow to be performed (claim 18).

Claim 19 relates to sampling and in particular analyzing a sample of the cell culture while keeping the connection to the virtual identity.

A bioprocess may include adding additional materials, for example magnetic beads for selection, to a cell culture. Claim 20 describes how those additional materials may be tracked and connected to the virtual identity.

Another teaching according to claim 21, which is of equal importance, relates to a control unit adapted to gather the data in the proposed method.

All explanations given with regard to the proposed method are fully applicable.

In the following, embodiments of the invention are explained with respect to the drawing. The drawing shows in
- Fig. 1,: two embodiments of integrated bioprocessing systems,
- Fig. 2,: a path of the virtual identity through the integrated bioprocessing system of Fig. 1a) and a unit operation station in an enlarged view and
- Fig. 3,: a path of the virtual identity through the integrated bioprocessing system of Fig. 1b) and the assembly of a container with an adapter for the integrated bioprocessing system.

The integrated bioprocessing system 1 shown in the drawings is preferably adapted to perform a bioprocess for the manufacturing of genetically modified T cells. Here, T cells are genetically modified to express a chimeric antigen receptor (CAR). Consequently, the term "CAR-T cells" describes T cells that have been genetically modified to express a CAR. The genetically modified CAR-T cells, which represent the product of the bioprocess, may be administered to a patient and used to start or resume cancer treatment in the patient. As the bioprocess is performed, the initial immune cell culture is gradually processed. All explanations given are mainly directed to such a bioprocess. It may be pointed out, however, that those explanations are fully applicable to other bioprocesses as well.

The term "liquid immune cell culture" is to be understood in a broad sense and refers to an immune cell culture comprising at least one type of immune cells suspended as particles in any type of liquid. As will be explained below, the liquid immune cell culture may comprise other cell types that are not immune cells. Hence, the term "liquid immune cell culture" refers to a liquid immune cell culture at any stage during the bioprocess. Consequently, the type and fraction of immune cells present in the liquid immune cell culture will change during the bioprocess applied as certain immune cells are enriched or depleted from the liquid immune cell culture and/or the immune cells are genetically modified.

The term "immune cells" generally refers to different types of white blood cells. Hence, the term "immune cells" includes a variety of cells, for example, but not limited to dendritic cells, T lymphocytes, also referred to as T cells, B lymphocytes, natural killer cells, macrophages or the like. Immune cells may also include subtypes of immune cells, for example tumor-infiltrating lymphocytes or different types of T cells. Subtypes of a certain type of immune cells may be classified based on the type of antigen present at the cell surface. Hence, the term immune cells may for example refer to T cells comprising the surface antigen CD4 ("CD4+ T cells"). Typically, a certain type of immune cells, e.g., T cells, preferably a certain subtype of immune cells, e.g., CD4+ T cells, will be selectively enriched by the bioprocess, while other immune cells, e. g. macrophages, and/or other cell types that are not immune cells, e. g., erythrocytes, and/or other subtypes of immune cells, e.g., CD8+ T cells, will be depleted from the liquid immune cell culture. The immune cells to be enriched are referred to as target immune cells, all other components to be depleted from the liquid immune cell culture are referred to "impurities". Further, and as mentioned above, the target immune cells might be genetically modified.

The term "naive cells" refers to cells that can still differentiate into different target cell types. In particular, stem cells and their derivatives prior to full differentiation into a specific cell type are naive cells. The term also comprises naive immune cells.

The term "liquid" is to be understood in a broad sense as well and refers to any liquid and/or particle-containing liquid that is processed within the integrated bioprocessing system 1. Hence, the term liquid might refer to media, waste, the liquid immune cell culture, byproducts obtained during the bioprocess, samples and/or an initial immune cell culture.

Preferably, an initial immune cell culture is obtained in a process called "leukapharesis". In leukapharesis, immune cells are obtained from the patient. Additionally or alternatively, the initial immune cell culture might also be obtained from a tissue of the patient. The initial immune cell culture might also be obtained by one or more donors that are not the patient.

Depending on the source of the initial cell culture and the bioprocess to be carried out, the initial cell culture, particularly the type, amount and distribution of impurities as well as target immune or naive cells might vary.

Presently, it is preferred that any initial immune cell culture used in the bioprocess is used for only a single patient or a small number of patients, for example up to ten patients. If that is not the case then the bioprocess presently described will yield only a cell culture for a single patient or a small number of patients and cell cultures for other patients are derived from different bioprocesses. Therefore, the proposed integrated bioprocessing system 1 is used for small scale bioprocesses.

The integrated bioprocessing system 1 may perform unit operations of the processing step or processing steps "enrichment" and/or "selection" and/or "activation" and/or "loading" and/or "genetic modification" and/or "expansion" and/or "formulation" and/or "fill" and/or "wash" and/or "separation" on the cell culture.

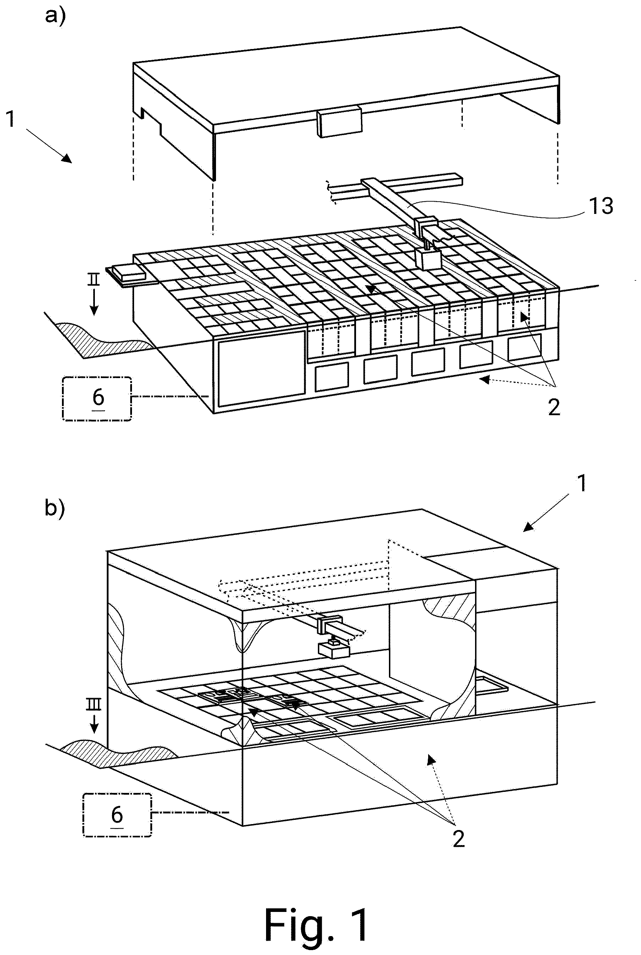

Fig. 1 shows two embodiments of integrated bioprocessing systems 1. The embodiment in Fig. 1a) is based on unit operation stations 2 adapted to perform one or more unit operations on fluidic structures inside of cartridges. The cartridges are containers 3 as will be described. The embodiment of Fig. 1b) is also cartridge-based but in this case the cartridges are connected via an adapter 4 to the integrated bioprocessing system 1. The adapter 4 allows having standardized interfaces at the integrated bioprocessing system 1 and individualizing the adapters 4 for use with, in particular single-use, cartridges to perform different unit operations. The adapters 4 may comprise individual drives, devices for the unit operations and the like which are driven via standardized interfaces of the integrated bioprocessing system 1 providing for example electrical energy and a data connection and possibly mechanical energy.

Proposed is a method for performing and supporting a bioprocess performed on a liquid immune or naive cell culture, wherein the bioprocess is performed on the cell culture to obtain a processed cell culture, wherein the processed cell culture is destined for autologous or allogenic cell therapy, wherein the bioprocess is performed by an integrated bioprocessing system 1 performing multiple unit operations on the cell culture.

A virtual identity 5 is assigned to the cell culture. The virtual identity 5 makes it possible to keep track of the cell culture. The virtual identity 5 may be a private cryptographic key, a globally unique identifier, a name or the like. The virtual identity 5 may also be a reference to another virtual identity 5 stored in a central memory 6.

The virtual identity 5 is stored by a control unit 7 in a local memory 8. The control unit 7 may be a local unit with a processor, possibly a user interface and the like. It may comprise a microprocessor, hardware logic like FPGAs or other suitable processors. The processor and the memory, possibly the control unit 7 as such, may be a single device.

The control unit 7 gathers data about the surroundings of the cell culture while the cell culture is processed by the integrated bioprocessing system 1 and the gathered data is stored assigned to the virtual identity 5.

The term "surroundings" is to be understood broadly. The gathered data may include sensor data about the actual space around the cell culture like an air temperature and/or an intensity of the light. The gathered data may also comprise data about other components acting on the cell culture, data about the bioprocess performed on the cell culture and the like.

The gathered data may be stored by the control unit 7 locally or sent to another control unit 7, a server, a control of the integrated bioprocessing system 1 or the like.

Essential is that the cell culture is transported from a first location to a second location and that the control unit 7 including the memory is transported with the cell culture from the first location to the second location.

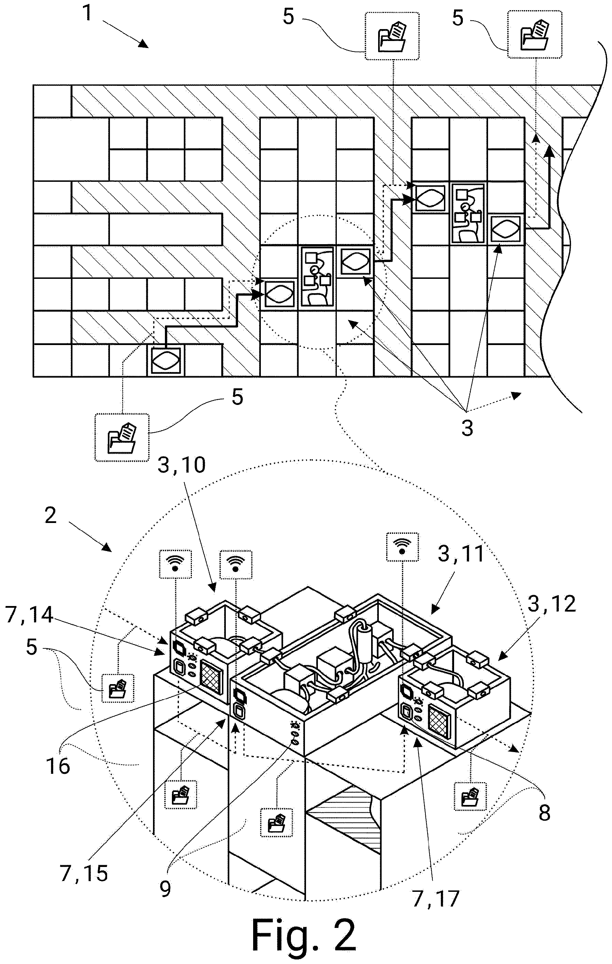

The cell culture may be transported by the integrated bioprocessing system 1, in particular autonomously. The transport may be through fluid connections like tube connections. The transport may also be a transport of a container 3 containing the cell culture. Fig. 2 shows in the enlarged potion a unit operation station 2. At this unit operation station 2, the integrated bioprocessing system 1 performs at least one unit operation of the bioprocess. Fig. 2 shows a first container 10, a second container 11 and a container containing additional material 12. Here and preferably the first container 10 and the second container 11 are used for containing the cell culture while the container containing additional material 12 is used to transport a fluid into the second container 11 which is mixed with the cell culture. Such a fluid may be a nutrient solution, a fluid containing magnetic beads for selection or the like.

In this case, a unit operation is performed inside the second container 11, which is a cartridge here, by the integrated bioprocessing system 1. Prior to that, the cell culture is transported to the unit operation station 2 in the first container 10 by a transport mechanism 13 shown in Fig. 1a). After the first container 10 with the cell culture has been transported to the unit operation station 2, which transport may well be proposed transport, tubes of the first container 10 and the second container 11 are here and preferably welded together. The cell culture is then pumped, so also transported, through the formed tube connection from the first container 10 to the second container 11. In this second transport, the control unit 7 is not transported with the cell culture. The virtual identity 5 however may be transported with the cell culture as will be explained. After the unit operation is performed, the cell culture may be pumped back into the first container 10 or into another container 3 (not shown). In the first case, the virtual identity 5 may well stay in the control unit of the first container 14, which may gather data about the unit operation.

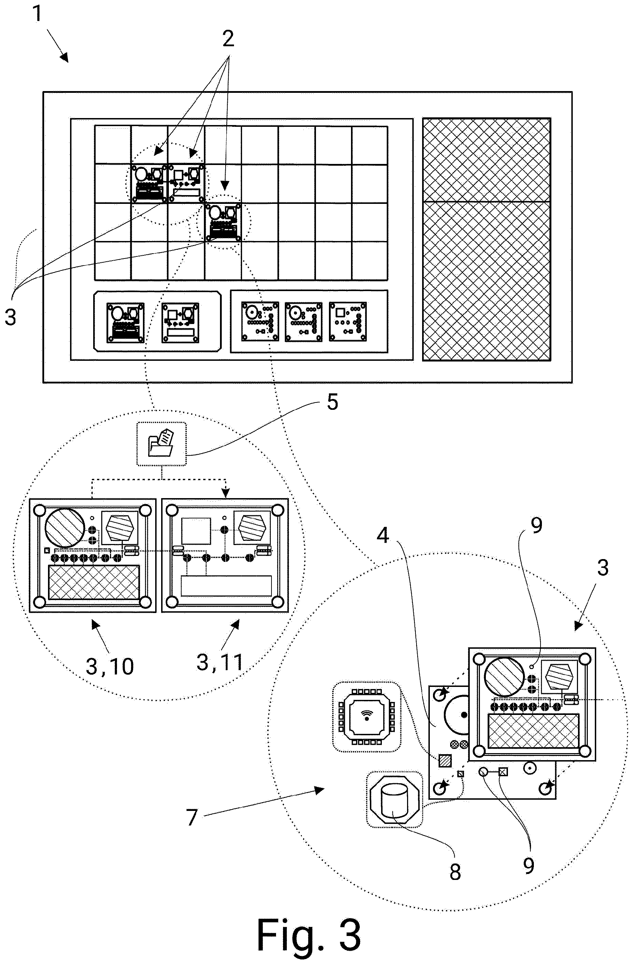

In Fig. 3, containers 3 are docked onto adapters 4 for the performance of unit operations. As can be seen, the control unit 7 may be part of the adapter 4.

It should be clear that the control unit 7 is not a central control unit 7 of the integrated bioprocessing system 1. The control unit 7 may gather the data at the first location and/or at the second location and/or during transport.

The cell culture may change its composition, may be derived from another cell culture and so on during the bioprocess. It may be genetically modified.

As can be seen in the figures, it may be the case, that the control unit 7 is placed at, in particular in, a container 3 of the integrated bioprocessing system 1 containing the cell culture and transported with the container 3, in particular by transporting the container 3. The control unit 7 may be embedded into the container 3 or may be placed in the container 3 or on the container 3.

Here and preferably the integrated bioprocessing system 1 autonomously transports the container 3 with the control unit 7 from the first location to the second location. Autonomously means that no user action on the container 3 is necessary.

According to one embodiment it is proposed, that the integrated bioprocessing system 1 transports the cell culture from the container 3 as a first container 10 to a second container 11. As has been explained, this may be done by pumping the cell culture through a fluid connection. The integrated bioprocessing system 1 may also grab a bag with the cell culture and move the bag, for example.

The virtual identity 5 is also transported from the first container 10 to the second container 11. Figs. 2 and 3 show a digital transfer of the virtual identity 5 between the containers 3. Every explanation given with regard to the container 3 may be true for the first container 10 and/or the second container 11. Naturally, the virtual identity may be transferred by copying it, by creating a link or reference to it or by creating a new link to a central virtual identity or the like.

Preferably, the first container 10 and the second container 11 each comprise a control unit 7 and the virtual identity 5 is transferred from the control unit of the first container 14 to the control unit of the second container 15 and stored in a local memory 8 of the control unit of the second container 15. This combination of digital and physical transports of the virtual identity 5 is adapted to the specifics of integrated bioprocessing systems 1 and can be flexibly adapted.

Additionally or alternatively, the control unit 7 including the memory is transported from the first container 10 to the second container 11, in particular autonomously by the integrated bioprocessing system 1.

In one embodiment, the integrated bioprocessing system 1 performs different unit operations on the cell culture at the first location and at the second location.

The transport of the control unit 7 may be a transport of the relevant part of the control unit 7, for example just the memory may be transported into a shell control unit 7 forming the control unit 7 out of the shell and the memory.

More in detail, it may be the case, that the integrated bioprocessing system 1 performs a transport routine for transporting the cell culture from the first container 10 to the second container 11. In the transport routine, the integrated bioprocessing system 1 may trigger the transfer of the virtual identity 5. Preferably, the integrated bioprocessing system 1 validates whether the transfer was successful before terminating the transport routine and continuing with the bioprocess. For example, the integrated bioprocessing system 1 may instruct the control unit of the first container 14 to initiate the transfer of the virtual identity 5 and afterwards query the control unit of the second container 15 whether it has received the virtual identity 5. The integrated bioprocessing system 1 may then compare the virtual identities 5, for example by having both control units 7 sign a transport protocol, to validate the transfer. After the validation, the integrated bioprocessing system 1 may continue with the bioprocess by further transporting the cell culture or acting on it and the control unit of the second container 15 may gather data about that. Additionally or alternatively, the transfer of the cell culture may be initiated only after the validation. Should the validation fail, the integrated bioprocessing system 1 may alert a user and may stop the bioprocess. A failed validation may be indicative of a defective second container 11.

According to one embodiment it is proposed, that the integrated bioprocessing system 1 transports the cell culture from the first container 10 to an intermediate container 3, in particular an intermediate container 3 without a control unit 7, and from the intermediate container 3 to the second container 11. For example, in a variation of the embodiment shown in Fig. 2, the first container 10 or a container 3 like the first container 10 could be an intermediate container 3 and the second container 11 of Fig. 2 could then be the first container 10. The intermediate container 3 may be used to transport the cell culture to another unit operation station 2 with a second container 11 like the second container 11 in Fig. 2. It may not be necessary to have a control unit 7 present for the intermediate transfer.

The virtual identity 5 is then transferred from the control unit of the first container 14 to the control unit of the second container 15 without being transferred to a control unit 7 of the intermediate container 3. Preferably, the integrated bioprocessing system 1 transports the intermediate container 3 with the cell culture.

In general, any explanations given with regard to the control unit 7 and for a container 3 may be true for any one, multiple of or all mentioned control units 7 or containers 3.

It is also possible that the control unit 7 comprises an encrypted and/or tamperproof component, in particular a trusted platform module. The virtual identity 5 may be stored encrypted in the control unit 7, and/or, the gathered data may be signed by the control unit 7, in particular with the virtual identity 5 and/or a private key placed in the encrypted and/or tamperproof component. In general, the control unit 7 may be used inside and outside of the integrated bioprocessing system 1. In particular outside, the control unit 7 may be protected against tampering. Further, the gathered data may be protected against forbidden changes, whether intentional or not, for example by the signing. Signing refers to cryptographical signing.

Preferably, the encrypted and/or tamperproof component comprises the memory and/or is transported with the control unit 7 to the second container 11.

According to one embodiment it is proposed, that the control unit 7 comprises a sensor 9 and/or is connected to a sensor 9, that the control unit 7 gathers data from the sensor 9 which is stored assigned to the virtual identity 5, preferably, that the cell culture is within sensor range, and/or, that the sensor 9 is a temperature sensor 9.

The sensor 9 may detect ambient changes and/or the presence of the cell culture and/or may monitor the performance of a unit operation. The sensor 9 may also be part of the integrated bioprocessing system 1 and connected to the control unit 7 temporarily. The sensor 9 may be controlled by the control unit 7 temporarily.

It is also conceivable, that the control unit 7 is connected to the integrated bioprocessing system 1, in particular to a component of a unit operation station 2 performing at least one unit operation on the cell culture. For example, the control unit 7 may connect to a centrifuge and monitor the centrifuges operating parameters. The control unit 7 may gather data from the connected component which is stored assigned to the virtual identity 5. Preferably, the component is directly involved in performing or controlling the unit operation. Additionally or alternatively, the component is a sensor control unit 7. Further, the gathered data can comprise sensor data from a sensor 9 involved in performing the unit operation and/or control data of the unit operation.

The control unit 7 may validate and/or store or send to storage, assigned to the virtual identity 5, a configuration of the component of the unit operation station 2.

According to one embodiment it is proposed, that the control unit 7 is connected via a short range wireless connection, in particular Bluetooth, to the component of the unit operation station 2, and/or, that the control unit 7 is connected via a data connection of an interface to the component of the unit operation station 2. Preferably, the container 3 is connected via the interface to the integrated bioprocessing system 1, more preferably, the integrated bioprocessing system 1 drives a component inside the container 3 to perform the unit operation via the interface, in particular mechanically and/or electrically drives a component inside the container 3.

For example, a, in particular single-use, component inside the container 3 may act directly on the cell culture. The component may be a centrifuge head, a pump head or a valve, for example. The integrated bioprocessing system 1, via the interface, may drive the centrifuge head or pump head or actuate the valve. Generally, the container 3 may be a single-use container 3 or contain a single-use fluidic structure.

Preferably, the gathered data is stored in a local memory 8 of the control unit 7, in particular in the same memory as the virtual identity 5. Equally preferred and in addition to that or alternatively, the data is stored in a central memory 6. The central memory 6 here and preferably is not part of the integrated bioprocessing system 1. The central memory 6 may be part of a data logger in accordance with Good Manufacturing Practice (GMP).

A user may predefine a template for the data storage of the gathered data. This enables automatically filling out an individualized report and makes documentation easier.

As has already been discussed, it may be the case that the control unit 7 including the memory is transported into the integrated bioprocessing system 1, and/or, that the control unit 7 including the memory is transported out of the integrated bioprocessing system 1. Preferably, the control unit 7 gathers data, in particular sensor data of the sensor 9 of the control unit 7, about the transport and/or a storage of the cell culture outside the integrated bioprocessing system 1.

According to one preferred embodiment it is proposed, that the virtual identity 5 is assigned to the cell culture when an initial cell culture is gathered from a patient, and/or, that the virtual identity 5 is transported, in particular with the control unit 7, to a location of an application of the cell culture to the patient. This may enable a physician or other party to check the manufacturing process inside the integrated bioprocessing system 1 even if not related to the entity operating the integrated bioprocessing system 1. Preferably, the virtual identity 5 is secured by the patient, in particular by biometrics of the patient. For example, the virtual identity 5 may be derived from biometric data of the patient. A physical token of the virtual identity 5 may be transported from the patient through the integrated bioprocessing system 1 back to the patient with the cell culture. In particular, the patient may authorize biometrically the physical token and at the end of the bioprocess when the processed cell culture arrives at the patient, the biometrics may be used to check that the cell culture is the correct cell culture. By having the physical token moving with the cell culture, the authenticity of the gathered data may be proven, at least to a greater extent.

Generally, not the whole control unit 7 has to be transported. Possibly, only the physical token, is transported, in particular through multiple containers 3. The physical token may be part of the control unit 7 or for example a separate chip with a private key or the like.

To provide redundancy, it may be the case, that at least some of the data gathered by the control unit 7 is independently gathered by the integrated bioprocessing system 1.

Here and preferably and as shown in Fig. 2, the control unit 7 comprises a display 16. Preferably, the control unit 7 displays at least some of the gathered data on the display 16, and/or, the bioprocess comprises a user action, in particular a user action on the cell culture. For that, the control unit 7 displays on the display 16 user guidance for the user action. User errors are very critical in a bioprocess performed on liquid immune or naive cell cultures. At the same time, user actions may be time critical. The display 16 allows reducing possible user errors.

Further, the control unit 7 may gather data about the user action which is stored assigned to the virtual identity 5. In this way, the control unit 7 may validate and/or document the user action. Documenting the user action may comprise documenting an identity of the user. The identity of the user may be detected biometrically and documented. Additionally or alternatively, the user may actively document that and/or how the user action has been performed, in particular may sign off the user action.

The control unit 7 may, additionally or alternatively, send user guidance to a user display 16, for example a wearable, a smartphone, smart glasses or the like.

According to one embodiment it is proposed, that the control unit 7 signs the gathered data or some of the gathered data, in particular a hash of the gathered data, together with a time stamp of a time base of the control unit 7 and/or of the integrated bioprocessing system 1 and/or together with some or all of the previously gathered data, in particular as a blockchain. Creating a trail of the gathered data related to a, in particular independent or tamper-proofed or otherwise trustworthy, time base makes it easier to detect problems with the gathered data. Preferably, the control unit 7 signs the gathered data or some of the gathered data together with some or all of the data gathered previously by another control unit 7 which had the virtual identity 5. That allows digitally tracing the physical path of the cell culture.

Further, a protocol of the transfer of the virtual identity 5 may be signed by both control units 7 and/or by the integrated bioprocessing system 1. Such double or even triple signing ensures that the transfer was performed correctly.

The integrated bioprocessing system 1 may not need to have all data about every bioprocess. Instead, the bioprocesses or some parts of the bioprocesses may be managed in a decentralized manner. The embodiment of Fig. 3 serves to show how an integrated bioprocessing system 1 may comprise standardized interfaces without the necessity of knowing exactly what those standardized interfaces drive. The actual unit operation can be defined and controlled by the adapter 4 and/or the container 3. It may be the case, that a workflow of the bioprocess is saved in the control unit 7. The control unit 7 may therefore be used for providing and monitoring the workflow.

Preferably, the control unit 7 analyzes the gathered data to detect a deviation from the workflow and initiates a deviation routine if it detects a deviation. The deviation routine may comprise informing a user and/or instructing the integrated bioprocessing system 1 to stop the bioprocess.

According to one embodiment it is proposed, that the control unit 7 sends at least part of the workflow to the integrated bioprocessing system 1 to inform the integrated bioprocessing system 1 of details of a next step in the bioprocess. Based on the received part of the workflow, the integrated bioprocessing system 1 may configure the unit operation station 2 or the interface or provide certain energy.

It may also be the case, that the integrated bioprocessing system 1 takes a sample from the cell culture, in particular for analysis. Data, in particular analysis data, about the sample may then be gathered and stored assigned to the virtual identity 5. Preferably, a copy of the virtual identity 5 is transferred with the sample to a sample processing station, in particular sample analysis station, and/or, the data is sent to the control unit 7 and stored assigned to the virtual identity 5 in the memory of the control unit 7. The virtual identity 5 may be transferred physically or virtually.

Here and preferably, at least one, preferably more than one, additional control unit 17 is transported with a container containing additional material 12 used in the bioprocess, in particular a liquid applied to the cell culture (Fig. 2). Preferably, data about the additional material, in particular data gathered by the additional control unit 17 about the surroundings of the additional material and/or data about the origin of the additional material, is stored assigned to the virtual identity 5. The data may be stored by the control unit 7 or the additional control unit 17. The additional control unit 17 may communicate data about the additional material, in particular data it gathered about the surroundings of the additional material and/or data about the origin of the additional material, to the control unit 7 and the communicated data may be stored assigned to the virtual identity 5, in particular by the control unit 7.

The additional material may be mixed with the cell culture. All explanations given with regard to the combination, handling and the like of the control unit 7 and the container 3 for the cell culture may apply to the additional control unit 17 for the container containing additional material 12. For example, the additional control unit 17 may receive data about the origin of the additional material and/or may be transported together with the additional material while gathering data about the surroundings of the additional material. In this way, it is possible to ensure that the additional material is in a good condition when applied to the cell culture and this good condition can also be documented by the additional control unit 17 or the control unit 7. Alternatively, the control unit 7 may communicate the virtual identity 5 to the additional control unit 17 and the additional control unit 17 may initiate central storing of its data.

Another teaching which is of equal importance relates to a control unit 7 adapted to gather the data in the proposed method.

## Claims

1. Method for performing and supporting a bioprocess performed on a liquid immune or naive cell culture, wherein the bioprocess is performed on the cell culture to obtain a processed cell culture, wherein the processed cell culture is destined for autologous or allogenic cell therapy, wherein the bioprocess is performed by an integrated bioprocessing system (1) performing multiple unit operations on the cell culture,
wherein a virtual identity (5) is assigned to the cell culture, wherein the virtual identity (5) is stored by a control unit (7) in a local memory (8), wherein the control unit (7) gathers data about the surroundings of the cell culture while the cell culture is processed by the integrated bioprocessing system (1) and wherein the gathered data is stored assigned to the virtual identity (5),
**characterized in**
**that** the cell culture is transported from a first location to a second location and that the control unit (7) including the memory is transported with the cell culture from the first location to the second location.

2. Method according to claim 1, **characterized in that** the control unit (7) is placed at, in particular in, a container (3) of the integrated bioprocessing system (1) containing the cell culture and transported with the container (3), in particular by transporting the container (3), preferably, that the integrated bioprocessing system (1) autonomously transports the container (3) with the control unit (7) from the first location to the second location.

3. Method according to claim 1 or 2, **characterized in that** the integrated bioprocessing system (1) transports the cell culture from the container (3) as a first container (10) to a second container (11), that the virtual identity (5) is transported from the first container (10) to the second container (11), preferably, that the first container (10) and the second container (11) each comprise a control unit (7), that the virtual identity (5) is transferred from the control unit of the first container (14) to the control unit of the second container (15) and stored in a local memory (8) of the control unit of the second container (15), and/or, that the control unit (7) including the memory is transported from the first container (10) to the second container (11).

4. Method according to claim 3, **characterized in that** the integrated bioprocessing system (1) performs a transport routine for transporting the cell culture from the first container (10) to the second container (11), that in the transport routine the integrated bioprocessing system (1) triggers the transfer of the virtual identity (5), preferably, that the integrated bioprocessing system (1) validates whether the transfer was successful before terminating the transport routine and continuing with the bioprocess.

5. Method according to claim 3 or 4, **characterized in that** the integrated bioprocessing system (1) transports the cell culture from the first container (10) to an intermediate container (3), in particular an intermediate container (3) without a control unit (7), and from the intermediate container (3) to the second container (11), that the virtual identity (5) is transferred from the control unit of the first container (14) to the control unit of the second container (15) without being transferred to a control unit (7) of the intermediate container (3), preferably, that the integrated bioprocessing system (1) transports the intermediate container (3) with the cell culture.

6. Method according to one of the preceding claims, **characterized in that** the control unit (7) comprises an encrypted and/or tamperproof component, in particular a trusted platform module, and/or, that the virtual identity (5) is stored encrypted in the control unit (7), and/or, that the gathered data is signed by the control unit (7), in particular with the virtual identity (5) and/or a private key placed in the encrypted and/or tamperproof component, preferably, that the encrypted and/or tamperproof component comprises the memory and/or is transported with the control unit (7) to the second container (11).

7. Method according to one of the preceding claims, **characterized in that** the control unit (7) comprises a sensor (9) and/or is connected to a sensor (9), that the control unit (7) gathers data from the sensor (9) which is stored assigned to the virtual identity (5), preferably, that the cell culture is within sensor (9) range, and/or, that the sensor (9) is a temperature sensor (9).

8. Method according to one of the preceding claims, **characterized in that** the control unit (7) is connected to the integrated bioprocessing system (1), in particular to a component of a unit operation station (2) performing at least one unit operation on the cell culture, that the control unit (7) gathers data from the connected component which is stored assigned to the virtual identity (5), preferably, that the component is directly involved in performing or controlling the unit operation, and/or, that the component is a sensor (9) control unit (7), more preferably, that the gathered data comprises sensor (9) data from a sensor (9) involved in performing the unit operation and/or control data of the unit operation.

9. Method according to claim 8, **characterized in that** the control unit (7) is connected via a short range wireless connection, in particular Bluetooth, to the component of the unit operation station (2), and/or, that the control unit (7) is connected via a data connection of an interface to the component of the unit operation station (2), preferably, that the container (3) is connected via the interface to the integrated bioprocessing system (1), more preferably, that the integrated bioprocessing system (1) drives a component inside the container (3) to perform the unit operation via the interface, in particular mechanically and/or electrically drives a component inside the container (3).

10. Method according to one of the preceding claims, **characterized in that** the gathered data is stored in a local memory (8) of the control unit (7), in particular in the same memory as the virtual identity (5), and/or, that the data is stored in a central memory (6), preferably, that the central memory (6) is not part of the integrated bioprocessing system (1).

11. Method according to one of the preceding claims, **characterized in that** the control unit (7) including the memory is transported into the integrated bioprocessing system (1), and/or, that the control unit (7) including the memory is transported out of the integrated bioprocessing system (1), preferably, that the control unit (7) gathers data, in particular sensor (9) data of the sensor (9) of the control unit (7), about the transport and/or a storage of the cell culture outside the integrated bioprocessing system (1).

12. Method according to one of the preceding claims, **characterized in that** the virtual identity (5) is assigned to the cell culture when an initial cell culture is gathered from a patient, and/or, that the virtual identity (5) is transported, in particular with the control unit (7), to a location of an application of the cell culture to the patient, preferably, that the virtual identity (5) is secured by the patient, in particular by biometrics of the patient, and/or, that a physical token of the virtual identity (5) is transported from the patient through the integrated bioprocessing system (1) back to the patient with the cell culture.

13. Method according to one of the preceding claims, **characterized in that** at least some of the data gathered by the control unit (7) is independently gathered by the integrated bioprocessing system (1).

14. Method according to one of the preceding claims, **characterized in that** the control unit (7) comprises a display (16), preferably, that the control unit (7) displays at least some of the gathered data on the display (16), and/or, that the bioprocess comprises a user action, in particular a user action on the cell culture, that the control unit (7) displays on the display (16) user guidance for the user action, more preferably, that the control unit (7) gathers data about the user action which is stored assigned to the virtual identity (5).

15. Method according to one of the preceding claims, **characterized in that** the control unit (7) signs the gathered data or some of the gathered data, in particular a hash of the gathered data, together with a time stamp of a time base of the control unit (7) and/or of the integrated bioprocessing system (1) and/or together with some or all of the previously gathered data, in particular as a blockchain, preferably, together with some or all of the data gathered previously by another control unit (7) which had the virtual identity (5).

16. Method according to one of claims 3 to 15, **characterized in that** a protocol of the transfer of the virtual identity (5) is signed by both control units (7) and/or by the integrated bioprocessing system (1).

17. Method according to one of the preceding claims, **characterized in that** a workflow of the bioprocess is saved in the control unit (7), preferably, that the control unit (7) analyzes the gathered data to detect a deviation from the workflow and initiates a deviation routine if it detects a deviation, more preferably, that the deviation routine comprises informing a user and/or instructing the integrated bioprocessing system (1) to stop the bioprocess.

18. Method according to claim 17, **characterized in that** the control unit (7) sends at least part of the workflow to the integrated bioprocessing system (1) to inform the integrated bioprocessing system (1) of details of a next step in the bioprocess.

19. Method according to one of the preceding claims, **characterized in that** the integrated bioprocessing system (1) takes a sample from the cell culture, in particular for analysis, that data, in particular analysis data, about the sample is gathered and stored assigned to the virtual identity (5), preferably, that a copy of the virtual identity (5) is transferred with the sample to a sample processing station, in particular sample analysis station, and/or, that the data is sent to the control unit (7) and stored assigned to the virtual identity (5) in the memory of the control unit (7).

20. Method according to one of the preceding claims, **characterized in that** at least one, preferably more than one, additional control unit (17) is transported with a container containing additional material (12) used in the bioprocess, in particular a liquid applied to the cell culture, preferably, that data about the additional material, in particular data gathered by the additional control unit (17) about the surroundings of the additional material and/or data about the origin of the additional material, is stored assigned to the virtual identity (5), more preferably, that the additional control unit (17) communicates data about the additional material, in particular data it gathered about the surroundings of the additional material and/or data about the origin of the additional material, to the control unit (7) and that the communicated data is stored assigned to the virtual identity (5).

21. Control unit adapted to gather the data in the method according to one of the preceding claims.
